# DEMANDE DE BREVET EUROPEEN

(11) **EP 1 108 400 A1**
(43) Date de publication de la demande: **20.06.2001**
(21) Numéro de dépôt: 99811143.9
(22) Date de dépôt: 13.12.1999
(51) Int. Cl.: A61F 2/04, A61F 2/06

(54) **Dispositif de fixation amovible d'une prothèse dans un conduit d'un organisme vivant**

(71) Demandeur: Biomedix S.A., 1701 Fribourg (CH)
(72) Inventeur: Godin, Norman, Dr., 1208 Geneve (CH)
(74) Mandataire: Savoye, Jean-Paul

(57) **Abrégé**

Ce dispositif de fixation amovible d'une prothèse de forme générale tubulaire (1) est destinée à contrôler le sens de l'écoulement dans un conduit (O) d'un organisme vivant, cette prothèse (1) étant en un matériau élastiquement déformable biocompatible solidaire d'une partie annulaire de fixation (la), l'introduction et l'enlèvement de cette prothèse (1) étant destinés à être réalisés en suivant ledit conduit (O). Il comporte des moyens élastiques annulaires (2, 4) conformés pour développer une force centrifuge très sensiblement supérieure à celle dudit matériau élastiquement déformable et destinés à appliquer ladite portion annulaire (la) de ladite prothèse (1) contre la paroi dudit conduit (O), ces moyens élastiques annulaires (2, 4) permettant une réduction sensible de leur section consécutivement à l'application d'une force centripète.

## Description

La présente invention se rapporte à un dispositif de fixation amovible d'une prothèse de forme générale tubulaire destinée à contrôler le sens de l'écoulement dans un conduit d'un organisme vivant, cette prothèse étant en un matériau biocompatible solidaire de l'organe annulaire de fixation, l'introduction et l'enlèvement de cet organe étant destinés à être réalisés en suivant ledit conduit.

De telles prothèses ont notamment été proposées pour contrôler le reflux gastrique dans l'oesophage chez des personnes souffrant généralement d'une hernie hiatale accompagnée d'une oesophagite de reflux. Ces reflux répétés d'acide gastrique attaquent la paroi de l'oesophage et créent finalement une oesophagite qui se traduit par une ulcération de la paroi et parfois un rétrécissement de sa section de passage. On a proposé, notamment dans le WO 96/29954 une prothèse destinée à permettre l'écoulement des aliments de l'oesophage vers l'estomac mais d'arrêter le reflux du contenu gastrique de l'estomac dans l'oesophage aux pressions de reflux en se repliant et/ou se "collapsant".

Le problème que posent de telles prothèses est celui de leur fixation et celui de leur changement donc aussi bien de leur mise en place que de leur enlèvement. En effet, cette affection étant chronique, un malade devra être muni constamment d'une telle prothèse, de sorte que celle-ci devra être changée périodiquement, notamment en raison du vieillissement des matières utilisées, généralement des élastomères, et des conditions particulièrement agressives du milieu dans lequel elle se trouve, l'acide gastrique ayant un pH de l'ordre de 1. Or, les seules solutions proposées sont soit chirurgicales, soit des éléments d'accrochage dont la nature n'est pas spécifiée. La voie chirurgicale ne serait acceptable à la rigueur que si la prothèse pouvait être garantie pour la durée de vie du patient. Or, on sait bien que tout élastomère vieillit et que le milieu dans lequel la prothèse est appelée à fonctionner est en plus particulièrement agressif, de sorte que la voie chirurgicale ne constitue pas une solution puisqu'on devrait y avoir recours périodiquement, alors qu'il existe d'autres voies thérapeutiques médicamenteuses comprenant les antiacides qui tendent à rendre neutre le milieu, les anti-histaminiques H2 qui se fixent sur les récepteurs H2 de la cellule pariétale ainsi que des médicaments qui bloquent la production des ions H+ par la cellule pariétale. Ces médicaments ont en commun de ne pas guérir le mal dont souffre le patient, de sorte qu'ils doivent être administrés de façon continue. Il est évident qu'à ce jour, aucune des solutions proposées n'est satisfaisante, dans la mesure où elles ne proposent qu'une alternative entre l'administration continue de médicaments et des interventions chirurgicales répétées.

Il est évident que la prothèse constitue sans doute la meilleure solution potentielle, dans la mesure où elle permet de remédier par des moyens purement mécaniques au dysfonctionnement dont souffre le patient. Cependant cette solution n'est envisageable qu'à partir du moment où la pose et l'enlèvement de cette prothèse pourraient être réalisées par voie endoscopique.

C'est précisément le but de la présente invention de proposer une solution qui permette la pose aussi bien que l'enlèvement de la prothèse par voie endoscopique.

A cet effet, la présente invention a pour objet un dispositif de fixation amovible d'une prothèse dans un conduit d'un organisme vivant selon la revendication 1.

Le dessin annexé illustre, schématiquement et à titre d'exemple, une forme d'exécution du dispositif de fixation objet de la présente invention.
La figure 1 est une vue en élévation d'une prothèse selon cette forme d'exécution;
la figure 2 est une vue en coupe selon II-II de la figure 1;
la figure 3 est une vue en perspective d'un détail du dispositif de fixation de la prothèse;
la figure 4 est une vue en perspective d'un autre détail du dispositif de fixation de la prothèse à l'état contracté;
la figure 5 est une vue en perspective du détail illustré par la figure 4,
la figure 6 est une vue en coupe de la base de l'oesophage et du haut de l'estomac avec la prothèse fixée par le dispositif de fixation selon l'invention.

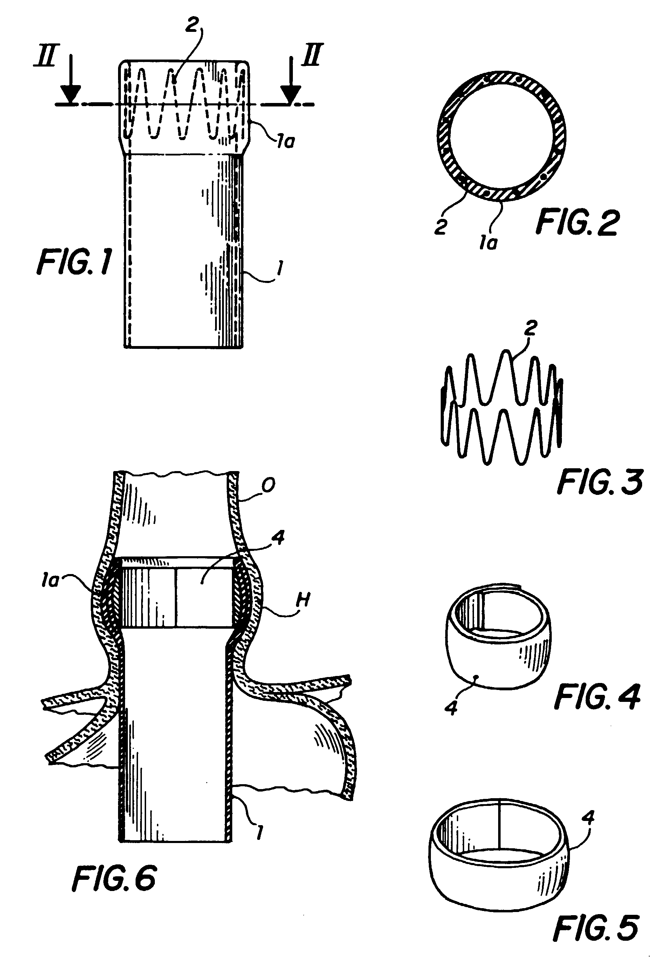

Sur la figure 1, une prothèse tubulaire 1 est représentée, venue d'une pièce par moulage avec une partie annulaire de fixation la. Cette prothèse 1 est destinée à empêcher le reflux oesophagien. Une telle prothèse est décrite en détail dans le WO 96/29954 auquel on pourra se référer pour plus de détails.

Cette prothèse tubulaire 1 est réalisée en un élastomère biocompatible, par exemple un élastomère à base de silicone à deux composants de qualité médicale, vendu sous la marque Silastic® par Dow Corning Corp, ou un silicone de la maison Nusil. On peut aussi utiliser des caoutchoucs de type butyle. Elle comporte à une extrémité une partie annulaire de fixation la.

Un élément élastique de renforcement 2 est noyé dans la partie annulaire de fixation la. Cet élément élastique 2 doit exercer une force centrifuge destinée à appliquer la partie annulaire de fixation la contre la paroi du conduit de l'organisme dans lequel il doit prendre place, dans cet exemple ce conduit étant soit l'oesophage, soit plus vraisemblablement la hernie hiatale que présentent la plupart des patients souffrant de reflux gastrique chronique, comme illustré par la figure 6. Cet élément élastique de renforcement 2 doit en même temps être suffisamment rétractable consécutivement à l'application d'une force centripète pour permettre de réduire très sensiblement sa section en vue de son insertion dans le conduit de l'organisme vivant dans lequel il doit fixer la prothèse tubulaire 1 par voie endoscopique ainsi que pour son retrait dudit conduit.

A cet effet, l'élément élastique de renforcement peut prendre différentes formes, telle que celle illustrée par la figure 3 dans laquelle l'élément élastique de renforcement est constituÈ par un fil à ressort 10, disposé en un anneau d'une certaine largeur formant des méandres réguliers d'un bord à l'autre de l'anneau. Cet élément élastique 2 peut être fabriqué par exemple en alliage à mémoire de forme en nickel-titane permettant une forte déformation lors de la pose et du retrait, ou en acier ressort de qualité médicale. Un élastomère dans lequel l'élément élastique 2 est noyé est biocompatible et protège la paroi des tissus de toute blessure due à l'élément élastique et empêche le déplacement de la prothèse.

L'élément élastique de renforcement 2 peut être fortement déformé par une force centripète afin de réduire sa section en vue de lui permettre d'être introduit par voie endoscopique.

Comme illustré par la figure 6, la prothèse 1 est fixée à la base de l'oesophage O, en général dans une partie formée par une hernie hiatale H. La partie de la prothèse 1 utilisée pour sa fixation est la partie annulaire la dans laquelle l'élément élastique de renforcement 2 est noyé. On voit sur cette figure 6 qu'une bague fendue 4 est disposée à l'intérieur de la partie annulaire de fixation 1a.

Cette bague fendue 4 est une bague élastique, susceptible de s'enrouler en spirale, comme illustré par la figure 4, lorsqu'une pression centripète lui est appliquée. En la libérant, elle reprend sa section initiale comme illustré par la figure 5. Pour lui permettre d'épouser la forme de la hernie, sa face externe peut avantageusement être bombée, comme on le voit en particulier sur les figures 4 et 5. Il est évidemment également possible d'utiliser une lame-ressort d'égale épaisseur et de la cintrer dans sa largeur.

La bague élastique fendue 4 permet en choisissant une dimension appropriée au diamètre de la hernie hiatale H, d'exercer une pression centrifuge sur la partie de fixation annulaire la de la prothèse 1, apte à maintenir cette prothèse en place avec suffisamment de force pour assurer son maintien en résistant au passage du bol alimentaire à travers elle. Le matériau utilisé pour réaliser la bague élastique peut être un métal, tel que de l'acier inoxydable, ou une matière plastique suffisamment rigide et élastique, ou encore un métal revêtu d'une matière plastique biocompatible.

La pose de la prothèse 1 utilisant la bague élastique fendue 4 s'effectue en deux temps, entièrement par voie endoscopique. Dans un premier temps, la prothèse souple 1 est repliée sur elle-même pour réduire son diamètre et permettre de l'introduire à travers l'oesophage O à l'aide d'un endoscope (non représenté), en positionnant la partie de fixation la dans l'hernie hiatale H ou à la base de l'oesophage O, si une telle hernie n'est pas formée, ce qui est rare dans ce genre d'affection chronique.

Une fois la prothèse 1 positionnée, on exerce une force centripète sur la bague fendue 4, par exemple à l'aide d'une pince ou d'un organe d'enroulement du type utilisé par les horlogers pour mettre un ressort-spiral dans un barillet (non représenté). On descend la bague élastique fendue 4 à travers l'oesophage O jusqu'à la position désirée et on libère cette bague 4, qui tend à reprendre sa section initiale sous l'effet de son élasticité.

Grâce à la possibilité d'enrouler la bague élastique fendue 4 en spirale pour réduire son diamètre, la force susceptible d'être développée par une telle bague fendue 4 est supérieure à celle du fil métallique 2 noyé dans la partie de fixation annulaire la de la prothèse 1. Les dimensions de la bague fendue 4 peuvent être choisies, d'une part en fonction des dimensions de la hernie hiatale, d'autre part en fonction de la force que l'on désire obtenir en position de fixation de la prothèse 1 dans la hernie H.

Dans une variante non représentée, la bague élastique fendue 4 pourrait être logée dans un espace annulaire ménagé à l'intérieur de la partie de fixation annulaire la et remplacé ainsi l'élément élastique de renforcement 2 noyé dans cette partie de fixation annulaire la. Dans ce cas, il faut que l'espace annulaire destiné à recevoir la bague élastique fendue 4 permette l'enroulement de cette bague en spirale lorsqu'une pression centripète est appliquée à la partie de fixation annulaire 1a.

A cet effet, une extrémité de cet espace annulaire peut être fermée tandis que l'autre extrémité s'ouvre sur la face interne de la partie annulaire de fixation la. Grâce à cette conformation de l'espace annulaire, lorsqu'une pression centripète s'exerce sur cette partie de fixation annulaire la, une extrémité de la bague élastique fendue 4 bute contre l'extrémité fermée de l'espace annulaire, de sorte que son autre extrémité sort par l'extrémité de cet espace s'ouvrant sur la face interne de la partie de fixation annulaire la permettant de réduire le diamètre de la partie annulaire de fixation la de la prothèse 1. En relâchant la pression centripète, la bague fendue 4 se dilate pour tendre à reprendre son diamètre initial. Avantageusement, l'extrémité de la bague fendue, adjacente à l'extrémité fermée de l'espace annulaire ménagé dans la partie annulaire de fixation, pourrait être fixée à cette extrémité.

## Revendications

1. Dispositif de fixation amovible d'une prothèse de forme générale tubulaire (1) destinée à contrôler le sens de l'écoulement dans un conduit (O) d'un organisme vivant, cette prothèse (1) étant en un matériau élastiquement déformable biocompatible solidaire d'une partie annulaire de fixation (la), l'introduction et l'enlèvement de cette prothèse (1) étant destinés à être réalisés en suivant ledit conduit (O), caractérisé par le fait qu'il comporte des moyens élastiques annulaires (2, 4) conformés pour développer une force centrifuge très sensiblement supérieure à celle dudit matériau élastiquement déformable et destinés à appliquer ladite portion annulaire (la) de ladite prothèse (1) contre la paroi dudit conduit (O), ces moyens élastiques annulaires (2, 4) permettant une réduction sensible de leur section consécutivement à l'application d'une force centripète.

2. Dispositif selon la revendication 1, caractérisé par le fait que lesdits moyens élastiques annulaires (2, 4) sont constitués au moins partiellement par une bague élastique fendue (4).

3. Dispositif selon la revendication 2, caractérisé en ce que ladite bague fendue (4) est un élément indépendant de ladite prothèse rapporté à l'intérieur de ladite partie annulaire de fixation (1a).

4. Dispositif selon l'une revendications précédentes, caractérisé par le fait que ladite prothèse (1) est en élastomère lesdits moyens élastiques étant constitués par au moins un ressort (2) noyé dans l'élastomère de ladite prothèse (1).

5. Dispositif selon la revendication 4, caractérisé par le fait que ledit ressort (2) est un fil sans fin, formant une série de méandres réguliers s'étendant d'un bord à l'autre d'un anneau.

6. Dispositif selon l'une des revendications précédentes, caractérisé en ce que ladite partie annulaire de fixation (1a) renferme un logement annulaire dont une extrémité est fermée et l'autre ouverte sur la face interne de ladite partie annulaire de fixation (1a) pour recevoir ladite bague élastique fendue (4).
